# EUROPEAN PATENT SPECIFICATION

(11) **EP 3 160 422 B1**
(45) Date of publication and mention of the grant of the patent: **14.08.2019**
(21) Application number: 15845889.3
(22) Date of filing: 25.06.2015
(51) Int. Cl.: A61K 6/033, A61L 27/12, C01B 25/32, A61F 2/28, C04B 28/34

(54) **NEW SINGLE-STEP MANUFACTURING PROCESS FOR FOAMED BIOMATERIALS**
NEUES EINSTUFIGES VERFAHREN ZUR HERSTELLUNG GESCHÄUMTER BIOMATERIALIEN
NOUVEAU PROCÉDÉ DE FABRICATION EN UNE SEULE ÉTAPE DE BIOMATÉRIAUX EN MOUSSE

(30) Priority: 25.06.2014 ES 201430964
(43) Date of publication of application: 03.05.2017
(73) Proprietor: Universitat Politècnica De Catalunya, 08034 Barcelona (ES)
(72) Inventor: PASTORINO CARRAZ, David, 08034 Barcelona (ES); CANAL BARNILS, Cristina, 08034 Barcelona (ES); GINEBRA MOLINS, Maria Pau, 08034 Barcelona (ES)
(74) Representative: Juncosa Miró, Jaime
(86) International application number: PCT/IB2015/001241
(87) International publication number: WO 2016/051244

(56) References cited:
- WO-A1-01/41821
- WO-A2-2005/077049
- WO-A2-2010/116321
- GB-A- 2 408 505
- US-A- 5 820 632
- US-A1- 2006 110 422
- US-A1- 2014 012 271
- US-B1- 6 340 648
- SARDA S ET AL: "Influence of surfactant molecules as air-entraining agent for bone cement macroporosity", JOURNAL OF BIOMEDICAL MATERIALS RESEARCH. PART A, WILEY PERIODICALS INC, HOBOKEN, NY, US, vol. 65A, no. 2, 1 January 2003 (2003-01-01), pages 215-221, XP002546856, ISSN: 1549-3296, DOI: 10.1002/JBM.A.10458
- MONTUFAR, E. B. ET AL.: 'Foamed surfactant solutionas a template for self-setting injectable hydroxyapatitescaffolds for bone regeneration.' ACTA BIOMATERIALIA vol. 6, no. 3, 2010, pages 876 - 885, XP026879128
- MONTUFAR, EDGAR B. ET AL.: 'Comparison of a lowmolecular weight and a macromolecular surfactant as foaming agents for injectable self setting hydroxyapatite foams: Polysorbate 80 versus gelatine.' MATERIALS SCIENCE AND ENGINEERING: C vol. 31, no. 7, 2011, pages 1498 - 1504, XP028272958

## Description

A new single-step manufacturing process for foamed biomaterials is described. Such process is based on the simultaneous mixing and foaming of a powder phase and a liquid phase to produce a macroporous structure.

### Technical field

This invention relates to processes for the preparation of biomaterials, in particular foams, scaffolds and solid structures, suitable for bone surgery and odontology, bone regeneration, bone defect fillings, stabilizing bone fractures, coating of prostheses or implants, fixing of prostheses or implants, drug delivery systems, and tissue engineering scaffolds, and to the biomaterials obtained thereby.

### Background

This invention is set in the area of biomaterials for bone tissue regeneration and, more specifically, in the area of calcium phosphate cements. Since 1985, when Brown and Chow submitted the first patent for this type of materials (US4518430), different formulations of calcium phosphate based cements have been developed. This type of materials are based on the mixture of one or more calcium salts with an aqueous solution to obtain a cement that is capable of setting in physiological conditions. The reaction product is a calcium phosphate with a composition very similar to the mineral phase forming the bone tissue. During setting, the plastic paste forms a solid structure.

The setting of the cement is a result of the dissolution process of the reagents and the precipitation of a new phase, which occurs at room- or body-temperature. In most of the cements developed during the last years, the reaction product is a hydroxyapatite, which is very similar to the biological one: it is nanocrystalline, non-stoichiometric and it can incorporate different ions depending on the composition of the reagents and the medium.

The development of cements, especially for treating bone defects, is associated with two important advantages in comparison with the use of calcium phosphates in granules or blocks. On the one hand, the injectability permits implanting of the cement via minimally invasive surgical techniques. On the other hand, the cement adapts perfectly to the geometry of the defect, even in complicated ones, which secure a perfect apposition bone tissue-material. This allows the stabilization of the defect and the healing process is therefore faster.

The setting properties and the hardening of the cement can be adjusted by modifying different processing parameters, such as the chemical composition of the reagents, the particle size, the addition of seed materials, etc. This makes calcium phosphate cements very versatile materials, since they are able to adapt to clinical requirements for different applications.

Different studies have shown that calcium phosphate cements are extremely biocompatible, osteoconductive and that they stimulate bone regeneration. However, besides being osteoconductive, the ideal cement should be capable of being resorbed with the same velocity as the bone tissue can grow with, being progressively replaced by the newly formed bone tissue. Even though the majority of the developed cements are more resorbable than the high temperature sintered apatites, they present excessively slow resorption kinetics and in many cases the cement remains intact in the surrounding bone tissue during years.

Within the apatitic cements, which reabsorb mostly by active cellular resorption processes, the macroporosity plays an important role. These active resorption processes are based in the fact that the osteoclasts (bone cells) degrade the material layer by layer starting with the exterior bone-cement interface and going towards the interior. This process is very slow when it comes to a dense material or a microporous one. Indeed, different studies established that for the colonization process of the bone tissue to be possible, it is necessary to have pores bigger than 70µm. The conventional calcium phosphate cements, although micro- and nano- porous after the setting, do not contain macroporosity in this range that stimulates bone growth. This obstructs its resorption and its transformation into bone tissue in an adequate time period. On the contrary, if a material with an interconnected macroporosity is obtained, the angiogenesis and the tissue growth inside the material are permitted. If the macrophages and the osteoclasts can penetrate towards the interior of the material, the resorption will occur in volume and therefore it will be faster. On the other hand, the tissue ingrowth can be stimulated by filling the pores with osteoinductive or osteoconductive factors such as the bone morphogenetic proteins (BMPs). These factors are well known to a person skilled in the art. Other therapeutic agents as antibiotics or other drugs can also be introduced in the material, mixing them with the liquid phase or the powder phase.

When calcium phosphate cements set, they form a mesh of calcium phosphate crystals, formed by precipitation from the reagents solution. This structure has a very high porosity, within the micro or nanometric range, that may reach values up to a 60%. Nevertheless, this micro or nanoporosity is not enough to allow either the growth of the bone tissue towards the interior of the material, or the angiogenesis, being this aspect crucial in order to obtain real bone regeneration. This regeneration demands the progressive colonization of the material by newly formed tissue, and the simultaneous reabsorption of the material, by the action of the osteoclastic cells.

Patents EP 1150722 and US 6642285 disclose porous implants based on calcium phosphate cements, which are obtained in a two-step process, namely by obtaining a calcium-containing cement paste and subsequently mixing it with a hydrophobic liquid. A surface active agent is disclosed as an additive in the aqueous phase, with the purpose of lowering the surface tension and thus facilitating the formation of the emulsion between the two phases (the cement paste and the hydrophobic liquid). Choosing the hydrophobic liquid and the corresponding proportions adequately, the setting of the cement gives a macroporous hydroxyapatite structure.

Patent EP1117626 B2 discloses a method to produce foamed ceramics using an organic binder and/or surfactant(s) in a two-step process. First, the ceramic slip including the organic binder and optionally surfactant(s) is foamed by using a ball mill. The resulting foam is then heated to burn out the organic binder and sintered at high temperature, and therefore it is not suitable for injection into a mammal body and subsequent setting inside the mammal body.

Patent US6340648 B1 discloses a three-step process to produce macroporous bone substitutes. A slurry is prepared by dispersing/dissolving a calcium phosphate powder, an organic polymer and a foaming agent. Said slurry is then mechanically foamed and cross-linked to finally be dried and sintered at high temperature.

Patent US6713420 B2 discloses a process to produce porous ceramics with at least 80% of the pores presenting a diameter of at least 5 µm. The process consists of foaming a mixture of a crosslinkable polymeric resin, hydroxyapatite powder and a surfactant. A cross-linking product is then added thereto, and the foamed slurry is dried and sintered at high temperature, therefore not being suitable for injection into a mammal body and subsequent setting inside the mammal body. Similarly, Patent US 6340648 B1 describes a method for producing a calcium phosphate porous sintered body. The method comprises different steps; preparing a slurry by dispersing and/or dissolving calcium phosphate powder and an organic polymer material and a foaming agent; foaming it to a prescribed volume by stirring and/or gas introduction; adding a cross-linking agent and/or a cross-linking initiator to the foamed slurry; mixing and hardening it by cross-linking polymerization to form a compact; and drying the compact followed by sintering. Therefore, as in the patent described previously, the final step is a thermal treatment to obtain a sintered porous body.

Zhou et al. (Preparation of high open porosity ceramic foams via direct foaming molded and dried at room temperature, J Eur Ceram Soc 2014:1-10) discloses a three step process using α-TCP to stabilize the foam produced before sintering.

Patent EP1787626 B1 discloses a two-step process, wherein a liquid phase is foamed and subsequently mixed with either the powder phase or a paste previously prepared. Similarly, Montufar et al. published interesting results (Montufar, E. B. et al. Foamed surfactant solution as a template for self-setting injectable hydroxyapatite scaffolds for bone regeneration. Acta Biomater. 6, 876-885 (2010); Montufar, E. B., Traykova, T., Planell, J. A. & Ginebra, M. P. Comparison of a low molecular weight and a macromolecular surfactant as foaming agents for injectable self setting hydroxyapatite foams: Polysorbate 80 versus gelatine. Mater. Sci. Eng. C 31, 1498-1504 (2011)) regarding the obtention of self-setting calcium phosphate foams. The manufacturing process was based in the preparation of a stable water-based foam by mechanical whipping of an aqueous solution containing surfactant molecules. In a second step this stable foam was subsequently mixed with a calcium phosphate powder phase. The foamed liquid was claimed to act as the template for the fabrication of the solid foam.

Patent US 2014012271 A1 discloses a process and a device for preparing a calcium phosphate foam comprising: mixing a) a first phase comprising water, propellant, and a stabilizing agent, and b) a second phase comprising one or more sources for calcium and/or phosphate, and initiating foaming during the mixing of the first and second phases. The propellant allows for the foaming of the first and the second phase when the at least two phases are mixed and the stabilizing agent supports the bubbles formed during foaming.

Kim et al. (Self-setting wet foams to porous ceramics by direct foaming, Journal of Ceramics Processing Research 2013:14-4*)* discloses a process where mechanical frothing is performed after mixing a colloidal suspension with Portland Cement. Moreover, use of lithium salts is detrimental to biocompatibility.

Gauckler et al. (Microstructural control of self-setting particle-stabilized ceramic foams, J.Am.Ceram.Soc. 2011:94-1*)* presented a similar protocol where the addition of cement powder and accelerant was followed by dispersion and foaming.

While macroporous bodies were shown to be obtained by various processes, most of them did not lead to injectable materials. When considering the material used as bone graft, injectability ensures that the material fills the whole defect, even in case of complex geometry. Moreover, injectable materials can be used in minimally invasive surgeries and thus reduce the morbidity of the operation.

The sintering step of the green body present in various processes to produce macroporous calcium phosphates requires reaching high temperatures during an adequate amount of time, generally a few hours. The processes may then be time-consuming and costly.

Multiple steps in the manufacturing process may be an obstacle to an efficient up-scaling of the process. The industrial applicability and cost efficiency of the implementation of two or three-steps preparation of macroporous materials is low compared to a one and single step method. A reproducible single-step process allows an easier industrialization of the manufacturing process.

### Summary of the invention

This invention relates to a process for obtaining self-setting calcium phosphate foams by comprising the step of simultaneously mixing and foaming of a powder phase and a liquid phase, wherein the powder phase comprises at least one calcium source and at least one phosphate source, and wherein the liquid phase comprises an aqueous solution. At least one of the phases contains at least one additive selected from the group consisting of foaming agents and surfactants. Specifically, this invention describes the adequate composition and process to obtain n
self-setting calcium phosphate foams in a reproducible and industrially applicable manner. Due to their self-setting capacity, it is not necessary to sinter the materials after foaming. These foams can be allowed to set to provide a solid body, e.g. by the use of a mold having the shape of the bone defect of interest. Alternatively, the foam may be injected into a mammal body, preferably a human body, and set therein, providing a macroporous solid structure of calcium phosphate, with a high interconnected macroporosity, produced during the foaming process. The macroporosity is superimposed to the inherent micro/ nanoporosity of the calcium phosphate walls.

The process according to the invention is defined in claim 1 and it allows obtaining highly reproducible interconnected macroporous calcium phosphate solid structures, which allow for the fast bone ingrowth and having an adequate resorption rate. This makes possible the progressive substitution of the material by newly formed bone tissue.

A process for the preparation of a solid structure, suitable for use in bone surgery and odontology, bone regeneration, bone defect fillings, stabilizing bone fractures, coating of prostheses or implants, fixing of prostheses or implants, drug delivery systems, and tissue
engineering scaffolds, may comprise a process according to the invention and a further step of allowing the foam to set, either after injection into a mammal body, or outside a mammal body.

The calcium phosphate foams developed can be used either by injecting them directly into the body, as bone regeneration materials, or by manufacturing pre-set solid structures, which can be implanted in a solid state or which can be used as scaffolds or substrates for tissue engineering.

### Detailed description of the invention

This invention deals with the production or preparation process of a macroporous self-setting calcium phosphate foam that can be injected and that is obtained by simultaneously mixing and foaming a liquid phase and a powder phase comprising at least one calcium source and at least one phosphate source. The way suggested is to simultaneously mix and foam the liquid phase and the powder phase. The addition of surfactant molecules or a foaming agent in the cement, as a way to stabilize the air bubbles in the paste, allows obtaining an interconnected macroporous foam. It has been verified that the simultaneous mixing and foaming of the liquid phase and powder phase produces a high macroporosity with a high degree of interconnection between the macropores. No propellant is needed for the foaming process.

Accordingly, the invention relates to a process as defined in claim 1 for the preparation of a self-setting calcium phosphate foam, characterized in that it comprises the step of simultaneously mixing and foaming a powder phase and a liquid phase, wherein the powder phase comprises at least one calcium source and at least one phosphate source, and wherein the liquid phase comprises an aqueous solution, and at least one of the phases comprising one or more additives selected from the group consisting of surfactants and foaming agents. That is, the foam may be obtained by a one-step process. This is clearly different from the processes described previously ((Montufar, E. B. et al. Foamed surfactant solution as a template for self-setting injectable hydroxyapatite scaffolds for bone regeneration. Acta Biomater. 6, 876-885 (2010); Montufar, E. B., Traykova, T., Planell, J. A. & Ginebra, M. P. Comparison of a low molecular weight and a macromolecular surfactant as foaming agents for injectable self setting hydroxyapatite foams: Polysorbate 80 versus gelatine. Mater. Sci. Eng. C 31, 1498-1504 (2011) and EP1787626 B1), which require obtaining a stable liquid foam before mixing it with a powder phase. The term "powder phase" refers to one or various solids, each of them in particulate or powder form, i.e. with a specific particle size distribution and a homogeneous composition.

In the frame of the present invention, when a particle size is indicated, the method of measurement employed is the laser diffraction method.

According to the invention, the simultaneous mixing and foaming of the two phases performed by the repetitive back-and-forth displacement of the phases between two containers through a narrow connection between them, one of the containers containing initially the powder phase and the other one the liquid phase. Preferred is the repetitive back-and-forth displacement of the phases between two syringes connected by the tip.

In the frame of the present invention, the term "surfactants and foaming agents" comprises both surfactants, which are compounds that lower the surface tension (or interfacial tension) between two liquids or between a liquid and a solid, and may act as detergents, wetting agents, emulsifiers, foaming agents, and dispersants, and foaming agents, which are materials that facilitate formation of foam, and may either be a surfactant or a blowing agent.

Preferred are surfactants having foaming properties. Preferred examples of "surfactants and foaming agents" are alkylbenzenesulfonates, lignin sulfonates, fatty alcohol ethoxylates, alkhylphenolethoxylates, isocyanate, sodium bicarbonate, titanium hydride or zirconium hydride.

A high variety of molecules can be used as surfactant agents, with an anionic, cationic or non-ionic character. However, given that the designed material should be suitable for being injected into a mammal body, preferably a human body, and more precisely in the bone tissue, it is convenient, and therefore preferred, to select a biocompatible surfactant. In this sense, non-ionic surfactants are of special interest, although other kinds of surfactants are suggested. Some examples are sorbitan esthers, such as sorbitan monooleate or sorbitan monopalmitate, polyoxysorbitan esthers, such as polyoxyethylene sorbitan monooleate (Tween 80, Polysorbate 80) and polyoxyethylene sorbitan monolaurate (Tween 20). All of them are approved for parenteral formulations of drugs.

Polymeric surfactants, such as those based in ethylene oxide - propylene oxide block polymers (Poloxamers, also known as Pluronics are of interest for these applications along with some phospholipids, like lecithines or sucrose esters.

In a preferred embodiment, the non-ionic surfactant is selected from sorbitan monooleate, sorbitan monopalmitate, polyoxyethylene sorbitan monooleate, polyoxyethylene sorbitan monolaurate, polyoxyethylene-polyoxypropylene copolymers, phosphatidylcoline (lecithin), phosphatidylethanolamide, phosphatidylserine, phosphatidylinositol, lisophosphatidylcoline, lisophosphatidylethanolamine, esfingomyeline, sucrose monolaurate, sucrose monooleate, and other sucrose esters.

According to a particular embodiment, the additive selected from the group consisting of surfactants and foaming agents is a non-ionic surfactant. Preferably, the surfactant is polyoxyethylene sorbitan monooleate.

In a further particular embodiment, the polyoxyethylene sorbitan monooleate is added in the liquid phase in a weight % between 0.1 and 10 %, with respect to the liquid phase. According to further particular embodiments, the surfactant is used in a weight % between 0.5 and 5.

In another particular embodiment, Poloxamer 407, a polymeric surfactant, is further added in the powder phase, in a weight % between 1 and 20% with respect to the powder phase.

Preferably, the ratio between the volume, given in mL, of liquid phase, and the weight of powder phase, given in grams (g), is comprised between 0.35 mL (liquid phase)/g (powder phase) and 0.90 mL(liquid phase)/g(powder phase). Even more preferably, the ratio is between 0.50 and 0.65mL/g.

The at least one calcium source is preferably selected from the group consisting of tetratracalcium phosphate, dicalcium phosphate anhydrous, dicalcium phosphate dihydrate, alpha-tricalcium phosphate, beta-tricalcium phosphate, monocalcium phosphate monohydrate, hydroxyapatite, calcium deficient hydroxyapatite, fluorapatite, amorphous calcium phosphate, calcium- sodium- and potassium- phosphate, calcium- and sodium- phosphate, calcium- and potassium- phosphate, calcium pyrophosphate, calcium carbonate, calcium sulphate, calcium sulfate hemihydrate, calcium oxide and calcium hydroxide.

The at least one phosphate source is preferably selected from the group consisting of tetratracalcium phosphate, dicalcium phosphate anhydrous, dicalcium phosphate dihydrate, alpha-tricalcium phosphate, beta-tricalcium phosphate, monocalcium phosphate monohydrate, hydroxyapatite, calcium deficient hydroxyapatite, fluorapatite, amorphous calcium phosphate, calcium- sodium- and potassium- phosphate, calcium- and sodium- phosphate, calcium- and potassium- phosphate, calcium pyrophosphate and phosphoric acid.

Even more preferably, a common source is used as calcium source and phosphate source, for example tetratracalcium phosphate, dicalcium phosphate anhydrous, dicalcium phosphate dihydrate, alpha-tricalcium phosphate, beta-tricalcium phosphate, monocalcium phosphate monohydrate, hydroxyapatite, calcium deficient hydroxyapatite, fluorapatite, amorphous calcium phosphate, calcium- sodium- and potassium- phosphate, calcium- and sodium- phosphate, calcium- and potassium- phosphate, and calcium pyrophosphate.

According to a particular embodiment, the liquid phase further comprises between 0.1 and 5 weight % of one or more of Na₂HPO₄, NaH₂PO₄, KH₂PO₄ and K₂HPO₄.

According to a further particular embodiment, the liquid phase further comprises between 0.5 and 15 weight %, with respect to the total weight of the liquid phase, of at least one oligomeric compound or polymer. Preferably, the oligomeric compound or polymer is selected from the group consisting of poloxamer, sodium alginate, hydroxypropyl methyl cellulose, hydroxyethyl cellulose, hydroxypropyl cellulose, methyl cellulose, hydroxyethyl starch, soluble starch, cyclodextrin, dextran sulphate, polyvinylpyrrolidone, quitosan, and hyaluronic acid.

The powder phase can also contain additives to act as seed materials, such as precipitated tricalcium phosphate or precipitated hydroxyapatite, which facilitate the nucleation of the precipitating phase in the foam. Therefore, in another particular embodiment, the powder phase comprises alpha tricalcium phosphate with a medium particle size inferior to 100 micrometers. Preferably, the medium particle size of the alpha tricalcium phosphate is inferior to 15 micrometers.

According to another particular embodiment, the powder phase comprises precipitated tricalcium phosphate, in a quantity inferior to 10 weight % with regard to the total weight of the powder phase.

In a further particular embodiment, at least one of the liquid phase and the powder phase further comprises one or more biologically active agents. Preferably, said biologically active agent is selected from the group consisting of growth factors, anti-cancerogenic substances, antibiotics, anti-inflammatories, hormones, pro-angiogenics and antioxidants.

The size of the pores can be controlled through different parameters, like the concentration of the added surfactant, the particle size of the powder phase and the type and concentration of additives. This is a key aspect to facilitate the angiogenesis and the colonization of the material by newly formed bone.

For the preparation of the foams, any mix and composition of calcium and phosphate source is possible. The final product of the set foam can vary from dicalcium phosphate dihydrate (Ca/P=1), calcium deficient hydroxyapatite (Ca/P between 1.33 and 1.67), octacalcium phosphate (Ca/P= 1.33), stoichiometric hydroxyapatite (Ca/P= 1.67) or carbonate apatite (Ca/P= 1.7). The main reagents for the powder phase include a source of calcium and a source of phosphate, which can be present as a unique component or as two or more components. Thus, a unique calcium phosphate can be the principal reagent of the powder phase, as a source of calcium and phosphate. As an alternative, two or more components can be present in the powder phase and each of them can contain calcium, phosphate or calcium and phosphate. Some of the interesting calcium phosphates can be selected between the following: tetratracalcium phosphate, dicalcium phosphate anhydrous, dicalcium phosphate dihydrate, alpha-tricalcium phosphate, beta-tricalcium phosphate, monocalcium phosphate monohydrate, hydroxyapatite, calcium deficient hydroxyapatite, fluorapatite, amorphous calcium phosphate, calcium-sodium- and potassium- phosphate, calcium- and sodium- phosphate, calcium- and potassium- phosphate, and calcium pyrophosphate. Other interesting components as calcium sources are the following: calcium carbonate, calcium sulphate, calcium sulfate hemihydrate, calcium oxide or calcium hydroxide. Among the sources of phosphate, all the soluble phosphates and the phosphoric acid can be mentioned. The reagents should set after mixing with the liquid phase, which is an aqueous solution. This requires a precise selection of the calcium phosphates, which form part of the powder phase, their proportions and their characteristics, as it is shown for instance by Chow and Takagi EP0721320 B1, and Constantz US Pat. 4880610, 5820632 and 6375935, among others.

The liquid phase comprises an aqueous solution that can incorporate one or more phosphate, carbonate or silicate ions in dissolution, which can act as accelerants or retarders of the setting reaction, and which can be obtained by dissolution of different compounds. Among the accelerants, Na₂HPO₄, NaH₂PO₄, KH₂PO₄, K₂HPO₄, can be mentioned. Preferably, the accelerant of the setting reaction is Na₂HPO₄ in a concentration between 1 and 5 weight % of the liquid phase.

The liquid phase can also comprise one or more oligomeric compounds or polymers either dissolved or in suspension in the liquid phase of the cement, such as sodium alginate, hydroxypropyl methyl cellulose, hydroxyethyl cellulose, hydroxypropyl cellulose, methyl cellulose, hydroxyethyl starch, soluble starch, cyclodextrin, dextran sulphate, polyvinylpyrrolidone, quitosan, and hyaluronic acid, to increase the injectability and the cohesion of the cement paste, avoiding the disintegration of the same as it is submerged in the physiological medium, as mentioned in Driessens et al. U.S. Pat. No. 6,206,957.

One or more additives selected from the group consisting of surfactants and foaming agents are included at least in one of the two phases, namely, the liquid or/and the powder phase.

Once foamed, the obtained mixture can be injected into a mammal body, preferably a human body, using a syringe with a diameter between 0.2 and 15 mm, after a time inferior to 60 minutes after mixing has elapsed, preferably inferior to 15. Alternatively, it can be introduced in a mould and allowed to set, preferably under physiological conditions until the solid structure is obtained.

Accordingly, the invention also refers, in a further aspect, to a process for the preparation of a solid structure suitable for use in bone surgery and odontology, bone regeneration, bone defect fillings, stabilizing bone fractures, coating of prostheses or implants, fixing of prostheses or implants, drug delivery systems, and tissue engineering scaffolds, comprising the steps ofa) obtaining a self-setting calcium phosphate foam according to the process as defined above and in the claims; and b) allowing the self-setting calcium phosphate foam to set
outside a mammal body.

A solid structure is obtained by this process.

Preferably, the solid structure has a total porosity measured by mercury immersion porosimetry comprised between 25 and 95 vol % and a macroporosity (percentage of pores introduced by the process) comprised between 2 and 80 vol %.

Preferably, the solid structure comprises macropores having a diameter comprised between 5 and 700 µm as determined by Optical and Scanning Electron Microscopy.

That is, the self-setting foam according to the present invention, after setting, allows obtaining a solid structure of a total porosity comprised between 25 and 95 vol% and a macroporosity comprised between 2 and 80 vol%. Said solid structure comprises macropores having a diameter comprised between 5 and 800 µm as determined by Optical and Scanning Electron Microscopy, preferably between 50 and 700 µm, which constitute a macroporosity superimposed to the intrinsic porosity of the set cement, which is in the micro and/or nanoscopic level.

### Description of the drawings

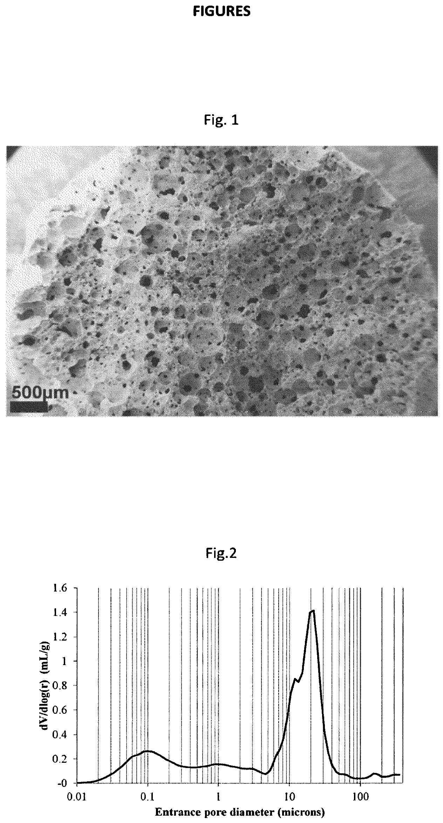

Fig. 1. As an illustrative example, a FESEM image of a section of the calcium phosphate foam obtained by a process which is not part of the invention is shown. The calcium phosphate foam is obtained by the addition of a 1 wt% of a biocompatible non-ionic surfactant to the liquid phase of an alpha-tricalcium phosphate cement, using a liquid to powder ratio of 0.55 ml/g and 10 wt% of Pluronic as additive in the powder phase. Mixing and foaming of the two phases was performed by mechanical whipping during 30 s at 6000 rpm.

In the following, the invention will be further illustrated with examples, although it must be understood that the examples should not be interpreted as restricting the invention to the compositions specified in the examples.

### EXAMPLES

**The examples 1-7 describe processes that are not part of the invention. Example 8 is part of the invention.**

### Examples 1-4

For the foam preparation, a liquid phase comprising 1 wt% of Tween80® (Polysorbate 80: polyoxyethylene (20) sorbitan monooleate or (x)-sorbitan mono-9-octadecenoate poly(oxy-1,2-ethanediyl) dissolved in distilled water was used. A powder phase comprising alpha-TCP particles including 2 wt% of precipitated hydroxyapatite as seed material was used. The liquid-to-powder ratio was chosen between 0.45 and 0.75 mL/g. The foams were prepared by simultaneously mixing and foaming the two phases, by mechanical whipping at 6000 rpm during 30 s, and were then moulded by injection into cylindrical moulds and allowed to set during approximately 24 hours, at 100 % relative humidity and 37 ºC, followed by 6 days submerged in distilled water at 37 ºC. The compressive strength, mean interconnection diameter and total porosity were then evaluated for each of the foams.

For all foams that were prepared, it was observed that an interconnected macroporous paste was obtained. Once submerged in distilled water, the calcium phosphate foams had cohesion, providing after setting solid foams of hydroxyapatite with different properties. The total porosity, interconnectivity and compressive strength are presented in Table 1.

**TABLE 1. Composition, compressive strength, total porosity and mean diameter of interconnection of the different foams prepared. The standard deviation is given within brackets (n=12).**

| **Example No.** | **L/P ratio (mL/g)** | **Compressive strength (MPa ± SD)** | **Total porosity (% ± SD)** | **Mean diameter of interconnections (µm ± SD)** |
|---|---|---|---|---|
| **1** | 0.45 | 1.87 (± 0.34) | 76.7 (± 1.1) | 123 ± 13 |
| **2** | 0.55 | 0.62 (± 0.06) | 83.9 (± 0.3) | 189 ± 41 |
| **3** | 0.65 | 0.29 (± 0.03) | 86.8 (± 0.6) | 203 ± 17 |
| **4** | 0.75 | 0.11 (± 0.02) | 89.0 (± 0.4) | 177 ± 19 |
| **Comparative Example** | 0.55 (EP1787626B1) | - | 71.98 (± 4.94) | - |

It was observed that, over the whole range of liquid to powder ratio studied, an effect on the macroporosity was obtained. In general, more porous foams were obtained when a higher liquid to powder ratio was used. The increase of the liquid to powder ratio reduced the compressive strength of the foams, and also produced a variation in the mean diameter of interconnection.

The coefficient of variation of foams with the same composition prepared via the protocol described in EP 1787626 B1 led to a variation of 6.86% while the protocol described herein led to a variation of 0.32 % in the value of the total porosity, as measured by mercury immersion and helium pycnometry.

### Examples 5-7

For the foam preparation, a liquid phase comprising 1 wt% of Tween80® dissolved in distilled water was used. A powder phase comprising alpha-TCP particles including 2 wt% of precipitated hydroxyapatite as seed material and a variable percentage of Pluronic F127 (Poloxamer 407), a polymeric surfactant based on ethylene oxide - propylene oxide block polymers was used. The liquid-to-powder ratio was fixed at 0.65 mL/g. The foam was prepared by simultaneously mixing and foaming the two phases, by mechanical whipping at 6000 rpm during 25 s and were then molded by injection into cylindrical moulds and allowed to set during approximately 24 hours at 100% relative humidity and 37 ºC, followed by 6 days submerged in distilled water at 37 ºC. The compressive strength, mean interconnection diameter and total porosity were then evaluated for each foam.

**Table 2:**

| Example No. | Pluronic (weight %) | Compressive strength (MPa ± **SD**) | Total porosity (%± **SD**) | Mean diameter of interconnections (µm ± SD) |
|---|---|---|---|---|
| **3** | 0 | 0.29±0.03 | 86.8±0.6 | 203 ± 17 |
| **5** | 5 | 0.22±0.02 | 88.9±1.1 | 143 ± 13 |
| **6** | 10 | 0.76±0.12 | 83.7±0.2 | 124 ± 21 |
| **7** | 15 | 0.86±0.07 | 84.72±1.25 | 97 ± 27 |

The addition of Pluronic F127 allowed controlling the diameter of the interconnections between the macropores from 203 µm down to 97 µm. The total porosity being superior to 80%, this additive allows tuning the pore size and mechanical properties.

### Example 8

For the foam preparation, a liquid phase comprising 1 wt% of Pluronic F-127 (Poloxamer 407) and 8 wt% of NaH₂PO₄ as accelerant dissolved in distilled water was used. A powder phase comprising alpha-TCP particles including 2 wt% of precipitated hydroxyapatite as seed material and 9 wt% of Pluronic F127 (Poloxamer 407) was used. The liquid-to-powder ratio was fixed at 0.55 mL/g.

The powder phase was placed in a 3mL syringe while the liquid phase was placed in a 5mL syringe. The two syringes were connected tip-to-tip using a double-female luer-lock connector. 10 back-and-forth movements were performed to simulatenously mix and foam the two phases, during 15 s. The foam was then moulded by injection into cylindrical molds and allowed to set during 7 d at 100 % relative humidity and 37 ºC.

The entrance pore size distribution as measured by mercury intrusion porosimetry is reported in Fig.2. The total porosity evaluated by mercury intrusion porosimetry is 74.0 %, 55.5 % of it being pores bigger than 10 µm.

## Claims

1. Process for the preparation of a self-setting calcium phosphate foam,
**characterized in that** it comprises a single step of simultaneous mixing and foaming a powder phase and a liquid phase, wherein the powder phase comprises at least one calcium source and at least one phosphate source, wherein the liquid phase is an aqueous solution, wherein the powder phase, the liquid phase or both contain at least one additive selected from the group consisting of surfactants and foaming agents, and wherein the mixing and simultaneous foaming are performed by back-and-forth movements of the material through a narrow connection between two containers, one of them initially containing the powder phase and the other one the liquid phase.

2. Process according to claim 1, **characterized in that** the simultaneous mixing and foaming are performed by mechanical whipping at a rotation speed between 1000 rpm and 15000 rpm.

3. Process according to claim 1, **characterized in that** the two containers are two syringes and the back-and-forth movements are performed through a tip-to-tip connection between the two syringes.

4. Process according to any one of claims 1 to 3, **characterized in that** at least one of the additives is a non-ionic surfactant and **in that** the surfactant is polyoxyethylene sorbitan monooleate.

5. Process according to any one of claims 1 to 4, **characterized in that** the polyoxyethylene sorbitan monooleate is added in the liquid phase at a weight % with respect to the liquid phase between 0.1 and 10 %.

6. Process according to any one of claims 1 to 5, wherein the ratio between mL of liquid phase and grams of powder phase is comprised between 0.35mL/g and 0.90mL/g.

7. Process according to any one of claims 1 to 6, wherein:
- the at least one calcium source is selected from the group consisting of tetratracalcium phosphate, dicalcium phosphate anhydrous, dicalcium phosphate dihydrate, alpha-tricalcium phosphate, beta-tricalcium phosphate, monocalcium phosphate monohydrate, hydroxyapatite, calcium deficient hydroxyapatite, fluorapatite, amorphous calcium phosphate, calcium- sodium- and potassium-phosphate, calcium- and sodium- phosphate, calcium- and potassium- phosphate, calcium pyrophosphate, calcium carbonate, calcium sulphate, calcium sulfate hemihydrate, calcium oxide and calcium hydroxide; and
- the at least one phosphate source is selected from the group consisting of tetratracalcium phosphate, dicalcium phosphate anhydrous, dicalcium phosphate dihydrate, alpha-tricalcium phosphate, beta-tricalcium phosphate, monocalcium phosphate monohydrate, hydroxyapatite, calcium deficient hydroxyapatite, fluorapatite, amorphous calcium phosphate, calcium- sodium- and potassium- phosphate, calcium- and sodium- phosphate, calcium- and potassium- phosphate, calcium pyrophosphate, and phosphoric acid.

8. Process according to any one of claims 1 to 7, **characterized in that** the liquid phase comprises between 0.1 and 5 weight % of one or more of Na₂HPO₄, NaH₂PO₄, KH₂PO₄ and K₂HPO₄.

9. Process according to any one of claims 1 to 8, **characterized in that** at least one of the phases comprises between 1 and 20 weight % of at least one oligomeric compound or polymer, wherein the oligomeric compound or polymer is selected from the group consisting of poloxamer, sodium alginate, hydroxypropyl methyl cellulose, hydroxyethyl cellulose, hydroxypropyl cellulose, methyl cellulose, hydroxyethyl starch, soluble starch, cyclodextrin, dextran sulphate, polyvinylpyrrolidone, quitosan, and hyaluronic acid.

10. Process according to any one of claims 1 to 9, **characterized in that** the powder phase comprises alpha tricalcium phosphate with a medium particle size inferior to 100 micrometers, preferably inferior to 15 micrometers.

11. Process according to any one of claims 1 to 10, **characterized in that** the powder phase comprises precipitated tricalcium phosphate or precipitated hydroxyapatite, in a quantity inferior to 10 weight % with regard to the total weight of the powder phase.

12. Process according to any one of claims 1 to 11, **characterized in that** at least one of the phases further comprises one or more biologically active agents, wherein the biologically active agent is selected from the group consisting of growth factors, anti-cancerogenic substances, antibiotics, and antioxidants.

13. Process for the preparation of a solid structure suitable for use in bone regeneration or tissue engineering, comprising the steps of
a) obtaining a self setting calcium phosphate according to the process as defined in any one of claims 1 to 12; and
b) allowing the self setting calcium phosphate foam to set, outside a mammal body.

## Patentansprüche

1. Verfahren zur Zubereitung eines selbstabbindenden Calciumphosphat-Schaumes, **dadurch gekennzeichnet, dass** es einen einzigen Schritt des gleichzeitigen Mischens und Schäumens einer Pulverphase und einer Flüssigphase umfasst, wobei die Pulverphase mindestens eine Calciumquelle und mindestens eine Phosphatquelle umfasst, wobei die Flüssigphase eine wässrige Lösung ist, wobei die Pulverphase, die Flüssigphase oder beide mindestens einen Zusatzstoff ausgewählt aus der Gruppe bestehend aus Tensiden und Schäummitteln enthalten, und wobei das Mischen und das gleichzeitige Schäumen mittels Hin- und Herbewegungen des Materials durch eine enge Verbindung zwischen zwei Behältern durchgeführt werden, wobei einer davon ursprünglich die Pulverphase enthält und der andere die Flüssigphase enthält.

2. Verfahren nach Anspruch 1, **dadurch gekennzeichnet, dass** das gleichzeitige Mischen und Schäumen mittels mechanischer Umrührung bei einer Rotationsgeschwindigkeit zwischen 1000 Umdrehungen pro Minute und 15000 Umdrehungen pro Minute durchgeführt werden.

3. Verfahren nach Anspruch 1, **dadurch gekennzeichnet, dass** die zwei Behälter zwei Spritzen sind und die Hin- und Herbewegungen durch eine Spitze-zu-Spitze-Verbindung zwischen den zwei Spritzen durchgeführt werden.

4. Verfahren nach einem der Ansprüche 1 bis 3, **dadurch gekennzeichnet, dass** mindestens einer der Zusatzstoffe ein nichtionisches Tensid ist und dass das Tensid Polyoxyethylen-Sorbitanmonooleat ist.

5. Verfahren nach einem der Ansprüche 1 bis 4, **dadurch gekennzeichnet, dass** das Polyoxyethylen-Sorbitanmonooleat der Flüssigphase bei einem Gew.-% in Bezug auf die Flüssigphase zwischen 0,1 und 10% hinzugefügt wird.

6. Verfahren nach einem der Ansprüche 1 bis 5, wobei das Verhältnis zwischen die ml der Flüssigphase und die Gramme der Pulverphase zwischen 0,35 ml/g und 0,90 ml/g liegt.

7. Verfahren nach einem der Ansprüche 1 bis 6, wobei:
- die mindestens eine Calciumquelle aus der Gruppe bestehend aus Tetratracalciumphosphat, Dicalciumphosphatanhydrid, Dicalciumphosphatdihydrat, alpha-Tricalciumphosphat, beta-Tricalciumphosphat, Monocalciumphosphatmonohydrat, Hydroxyapatit, calciumarmem Hydroxyapatit, Fluorapatit, amorphem Calciumphosphat, Calcium-, Natrium- und Kaliumphosphat, Calcium- und Natriumphosphat, Calcium- und Kaliumphosphat, Calciumpyrophosphat, Calciumcarbonat, Calciumsulphat, Calciumsulfathemihydrat, Calciumoxid und Calciumhydroxid ausgewählt wird; und
- die mindestens eine Phosphatquelle aus der Gruppe bestehend aus Tetratracalciumphosphat, Dicalciumphosphatanhydrid, Dicalciumphosphatdihydrat, alpha-Tricalciumphosphat, beta-Tricalciumphosphat, Monocalciumphosphatmonohydrat, Hydroxyapatit, calciumarmem Hydroxyapatit, Fluorapatit, amorphem Calciumphosphat, Calcium-, Natrium- und Kaliumphosphat, Calcium- und Natriumphosphat, Calcium- und Kaliumphosphat, Calciumpyrophosphat und Phosphorsäure ausgewählt wird.

8. Verfahren nach einem der Ansprüche 1 bis 7, **dadurch gekennzeichnet, dass** die Flüssigphase zwischen 0,1 und 5 Gew.-% eines oder mehrerer aus Na₂HPO₄, NaH₂PO₄, KH₂PO₄ und K₂HPO₄ umfasst.

9. Verfahren nach einem der Ansprüche 1 bis 8, **dadurch gekennzeichnet, dass** mindestens eine der Phasen zwischen 1 und 20 Gew.-% mindestens einer oligomeren Verbindung oder eines Polymeren umfasst, wobei die oligomere Verbindung oder das Polymer aus der Gruppe bestehend aus Poloxamer, Natriumalginat, Hydroxypropylmethylcellulose, Hydroxyethylcellulose, Hydroxypropylcellulose, Methylcellulose, Hydroxyethylstärke, löslicher Stärke, Cyclodextrin, Dextransulphat, Polyvinylpyrrolidon, Chitosan und Hyaluronsäure ausgewählt wird.

10. Verfahren nach einem der Ansprüche 1 bis 9, **dadurch gekennzeichnet, dass** die Pulverphase alpha-Tricalciumphosphat mit einer mittleren Partikelgröße kleiner als 100 Mikrometer, vorzugsweise kleiner als 15 Mikrometer, umfasst.

11. Verfahren nach einem der Ansprüche 1 bis 10, **dadurch gekennzeichnet, dass** die Pulverphase ausgefälltes Tricalciumphosphat oder ausgefälltes Hydroxyapatit, in einer Menge kleiner als 10 Gew.-% in Bezug auf das Gesamtgewicht der Pulverphase, umfasst.

12. Verfahren nach einem der Ansprüche 1 bis 11, **dadurch gekennzeichnet, dass** mindestens einer der Phasen zusätzlich ein oder mehrere biologisch aktive Mittel umfasst, wobei das biologisch aktive Mittel aus der Gruppe bestehend aus Wachstumsfaktoren, antikanzerogenen Substanzen, Antibiotika und Antioxidanten ausgewählt wird.

13. Verfahren zur Zubereitung einer festen Struktur, welche für dessen Verwendung bei der Knochenregeneration oder dem Gewebe-Engineering geeignet ist, umfassend die folgenden Schritte
a) das Erhalten eines selbstabbindenden Calciumphosphat nach dem Verfahren nach einem der Ansprüche 1 bis 12; und
b) das Erlauben des Abbindens des selbstabbindenden Calciumphosphatschaums außerhalb des Körpers eines Säugetiers.

## Revendications

1. Méthode pour la préparation de mousse de calcium de phosphate auto-durcissable, **caractérisée en ce qu**'elle comporte une seule étape de mélange et moussage simultanés d'une phase pulvérulente et une phase liquide, dans laquelle la phase pulvérulente comporte au moins une source de calcium et au moins une source de phosphate, dans laquelle la phase liquide est une solution aqueuse, dans laquelle la phase pulvérulente, la phase liquide, ou les deux, contiennent au moins un additif sélectionné du groupe consistant en des agents surfactants et moussants et dans laquelle le mélange et le moussage simultanés sont effectués par des mouvements de va-et-vient du matériau à travers une connexion étroite entre les deux conteneurs, l'un d'eux contenant initialement la phase pulvérulente et l'autre la phase liquide.

2. Méthode conformément à la revendication 1, **caractérisée en ce que** le mélange et moussage simultanés a lieu en fouettant à une vitesse de rotation de 1.000 t.p.m. à 1.500 t.p.m.

3. Méthode conformément à la revendication 1, **caractérisée en ce que** les deux conteneurs sont deux seringues et les mouvements de va-et-vient sont effectués à travers une connexion bout-à-but entre les deux seringues.

4. Méthode conformément à une quelconque des revendications 1 à 3, caractérisée **en ce qu**'au moins un des additifs est un surfactant non-ionique et **en ce que** le surfactant est du mono-oléate de sorbitane polyoxythyléné.

5. Méthode conformément à une quelconque des revendications 1 à 4 **caractérisée en ce que** le mono-oléate de sorbitane polyoxythyléné est ajouté à la phase liquide à un % du poids par rapport à la phase liquide entre 0,1 et 10%.

6. Méthode conformément à une quelconque des revendications 1 à 5 dans laquelle le ratio entre mL de phase liquide et grammes de phase pulvérulente est compris entre 0,35mL/g et 0,90mL/g.

7. Méthode conformément à une quelconque des revendications 1 à 6, dans laquelle :
- la au moins une source de calcium est sélectionnée du groupe consistant en du phosphate tétratracalcique phosphate dicalcique anhydre, phosphate calcique dihydraté, phosphate de tricalcium alpha, phosphate de tricalcium béta, phosphate monocalcique monohydraté, hydroxyapatite déficiente en calcium , fluorapite, phosphate calcique amorphe, phosphate calcique, sodique et potassique, phosphate calcique et sodique, phosphate calcique et potassique, pyrophosphate calcique, carbonate calcique, sulphate calcique, sulphate calcique hémihydraté, oxide calcique et hydroxide calcique ; et
- la au moins une source est sélectionnée du groupe consistant en phosphate tétratracalcique, phosphate dicalcique anhydre, phosphate dicalcique dihydraté, phosphate de tricalcium alpha, phosphate de tricalcium béta, phosphate monocalcique monohydraté, hydroxyapatite, hydroxyapatite déficiente en calcium, fluoropatite, phosphate calcique amorphe, phosphate calcique-sodique et potassique, phosphate calcique et sodique, phosphate calcique et potassique, pyrophosphate calcique et acide phosphorique

8. Méthode conformément à une quelconque des revendications 1 à 7, **caractérisée en ce que** la phase liquide comporte entre 0,1 et 5 % du poids d'un ou plusieurs Na₂HPO₄, NaH₂PO₄, KH₂PO₄ et K₂HPO₄.

9. Méthode conformément à une quelconque des revendications 1 à 8, **caractérisée en ce qu**'au moins une des phases comporte entre 1 et 20% du poids d'au moins un composé oligomérique ou du polymère, dans laquelle le composé oligomérique ou polymère est sélectionné du groupe consistant en du poloxamère, alginate de sodium, cellulose hydroxypropylméthyle, cellulose hydroxyéthyle, cellulose hydroxypropyle, cellulose méthyle, amidon hydroxyéthyle, amidon soluble, cyclodextrine, sulfate de dextrane, polyvynilpyrrolidone , chitosane et acide hyaluronique.

10. Méthode conformément à une quelconque des revendications 1 à 9 **caractérisée en ce que** la phase pulvérulente comporte du phosphate de tricalcium alpha ayant une taille de particule moyenne inférieure à 100 micromètres, de préférence inférieure à 15 micromètres.

11. Méthode conformément à une quelconque des revendications 1 à 9 **caractérisée en ce que** la phase pulvérulente comporte du -phosphate tricalcique précipité ou de l'hydroxyapatite précipitée dans une quantité inférieure à 10% du poids par rapport au poids total de la phase pulvérulente.

12. Méthode conformément à une quelconque des revendications 1 à 11 **caractérisée en ce qu**'au moins une des phases comporte par ailleurs un ou plusieurs agents biologiquement actifs, dans laquelle l'agent biologiquement actif est sélectionné du groupe consistant en des facteurs de croissance et des substance anti-cancérogènes, des antibiotiques et des antioxydants.

13. Méthode pour la préparation d'une structure solide pour son utilisation dans la technique de régénération des os ou des tissus, comportant les phases de
a) obtenir un phosphate de calcium de réglage automatique conformément à la méthode telle que définie dans une quelconque des revendications 1 à 12 ; et
b) permettre que la mousse de phosphate de calcium soit réglée automatiquement pour être réglée au dehors du corps d'un mammifère.
